# EUROPEAN PATENT APPLICATION

(11) **EP 2 835 135 A2**
(43) Date of publication of application: **11.02.2015**
(21) Application number: 14172834.5
(22) Date of filing: 17.06.2014
(51) Int. Cl.: A61K 38/16, A61P 31/04

(54) **Means and methods for treating pseudomonas infection**

(30) Priority: 19.06.2013 EP 13172741
(71) Applicant: Max-Planck-Gesellschaft zur Förderung der Wissenschaften e.V. Berlin, 80539 München (DE)
(72) Inventor: Grossmann, Tom N., 44137 Dortmund (DE); Ottmann, Christian, 44227 Dortmund (DE); Glas, Adrian, 44139 Dortmund (DE); Bier, David, 44227 Dortmund (DE)
(74) Representative: Vossius & Partner

(57) **Abstract**

The present invention relates to a peptide or depsipeptide of formula (I)

(Y)ₖX¹YZ¹X²Z²(Y)ₘ (I),

wherein X¹ is an α-amino acid residue or an α-hydroxy acid residue of formula (IIa)

-A-CR¹R²-CO- (IIa),

X² is an α-amino acid residue or an α-hydroxy acid residue of formula (IIb)

-A-CR³R⁴-CO- (IIb),

wherein A is NH or O; R¹ and R³ are independently selected from H, halogen such as F, substituted or unsubstituted C₁ to C₄ alkyl such as methyl, substituted or unsubstituted aryl and substituted or unsubstituted arylalkyl, aryl being a 5- or 6-membered ring with 0, 1, 2 or 3 heteroatoms, heteroatoms being selected from O, N and S, alkyl in said arylalkyl being C₁ to C₄ alkyl, said alkyl in said arylalkyl optionally being substituted, the term "substituted", when relating to R¹ and R³, providing for, as valence permits, 1, 2, 3, 4, 5, 6, 7 or 8 substituents, said substituents being chosen from halogen such as F; R² and R⁴ together are chosen to be substituted or unsubstituted n-alk-1,ω-diyl or substituted or unsubstituted n-alken-1,ω-diyl, ω being an integer number selected from 6, 7, 8, 9, 10, 11, 12, 13 and 14, the term "substituted", when relating to R² and R⁴, providing for 1 or 2, in case of the substituents being halogen 1, 2, 3, 4, 5, 6, 7 or 8 substituents, said substituents being chosen from halogen such as F and C₁ to C₄ alkyl such as methyl, said C₁ to C₄ alkyl in turn optionally being substituted with, as valence permits, 1, 2, 3, 4, 5, 6, 7 or 8 substituents, said substituents being chosen from halogen such as F, and the double bond in said n-alken-1,ω-diyl being at any one of positions 1, 2, 3, 4,... and (ω-1); each occurrence of Y is independently selected to be an α-amino acid or α-hydroxy acid; k is an integer number selected from 0, 1, 2, 3, 4, 5, 6 and 7; m is an integer number selected from 1, 2, 3 and 4; Z¹ is a polar or negatively charged α-amino acid or α-hydroxy acid; and Z² is a an aliphatic hydrophobic α-amino acid or α-hydroxy acid; the N-terminus of said peptide or depsipeptide is free; protected, preferably acetylated; or conjugated, preferably to a cell-penetrating peptide or a lipid; and the C-terminus of said peptide or depsipeptide is free; protected, preferably amidated; or conjugated, preferably to a cell-penetrating peptide or a lipid.

## Description

The present invention relates to a peptide or depsipeptide of formula (I)

(Y)ₖX¹YZ¹X²Z²(Y)ₘ (I),

wherein X¹ is an α-amino acid residue or an α-hydroxy acid residue of formula (IIa)

-A-CR¹R²-CO- (IIa),

X² is an α-amino acid residue or an α-hydroxy acid residue of formula (IIb)

-A-CR³R⁴-CO- (IIb),

wherein A is NH or O; R¹ and R³ are independently selected from H, halogen such as F, substituted or unsubstituted C₁ to C₄ alkyl such as methyl, substituted or unsubstituted aryl and substituted or unsubstituted arylalkyl, aryl being a 5- or 6-membered ring with 0, 1, 2 or 3 heteroatoms, heteroatoms being selected from O, N and S, alkyl in said arylalkyl being C₁ to C₄ alkyl, said alkyl in said arylalkyl optionally being substituted, the term "substituted", when relating to R¹ and R³, providing for, as valence permits, 1, 2, 3, 4, 5, 6, 7 or 8 substituents, said substituents being chosen from halogen such as F; R² and R⁴ together are chosen to be substituted or unsubstituted n-alk-1,ω-diyl or substituted or unsubstituted n-alken-1,ω-diyl, ω being an integer number selected from 6, 7, 8, 9, 10, 11, 12, 13 and 14, the term "substituted", when relating to R² and R⁴, providing for 1 or 2, in case of the substituents being halogen 1, 2, 3, 4, 5, 6, 7 or 8 substituents, said substituents being chosen from halogen such as F and C₁ to C₄ alkyl such as methyl, said C₁ to C₄ alkyl in turn optionally being substituted with, as valence permits, 1, 2, 3, 4, 5, 6, 7 or 8 substituents, said substituents being chosen from halogen such as F, and the double bond in said n-alken-1,ω-diyl being at any one of positions 1, 2, 3, 4,... and (ω-1); each occurrence of Y is independently selected to be an α-amino acid or α-hydroxy acid; k is an integer number selected from 0, 1, 2, 3, 4, 5, 6 and 7; m is an integer number selected from 1, 2, 3 and 4; Z¹ is a polar or negatively charged α-amino acid or α-hydroxy acid; and Z² is a an aliphatic hydrophobic α-amino acid or α-hydroxy acid; the N-terminus of said peptide or depsipeptide is free; protected, preferably acetylated; or conjugated, preferably to a cell-penetrating peptide or a lipid; and the C-terminus of said peptide or depsipeptide is free; protected, preferably amidated; or conjugated, preferably to a cell-penetrating peptide or a lipid.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The gram-negative bacterium *Pseudomonas aeruginosa* is a major cause of health-care associated infections, such as pneumonia and infections involving the urinary tract, wounds, burns, and the bloodstream (Rossolini et al., Clin. Microbiol. Infect., 11, 17-32 (2005)). Especially in compromised individuals like HIV patients and in cystic fibrosis *P. aeruginosa* infections are prevalent and are often accompanied by higher mortality (Talbot et al., Society of America. Clin. Infect. Dis., 42, 657-668 (2006)). Furthermore, corneal infection with *P*. *aeruginosa* is a major cause of visual impairment and blindness worldwide, occurring either as a result of trauma or in association with contact lens wear. Besides classical broad-spectrum antibiotic strategies, there is only a very limited number of therapeutic approaches. Examples include mimetics of natural membranolytic peptides (Srinivas et al., Science, 327, 1010-1013 (2010)) and anti-biofilm strategies (Bernardi et al., Chem. Soc. Rev., DOI: 10.1039/c2cs35408j (2013*)*)*.* Although infection with *P. aeruginosa* may be eradicated if treatment is commenced early, no current antibiotics are able to eradicate an established chronic *P. aeruginosa* infection involving multidrug-resistant strains (Talbot et al., Society of America. Clin. Infect. Dis., 42, 657-668 (2006)).

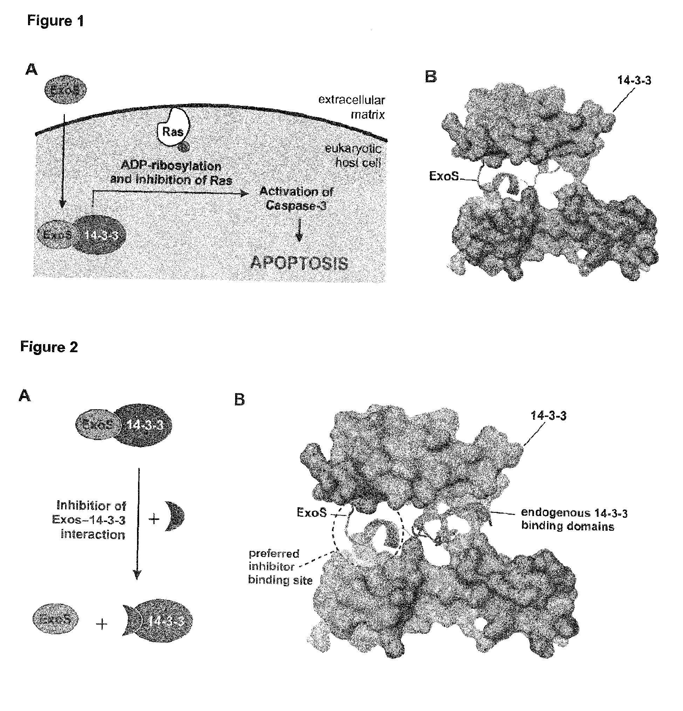

Like many other gram-negative bacteria, *P. aeruginosa* manipulates eukaryotic host cells by using a type III secretion system (T3SS) (Yahr et al., Mol. Microbiol., 22, 991-1003 (1996)). Despite extensive characterization, only four effector proteins of the *P. aeruginosa* T3SS have been identified: ExoS, ExoT, ExoU, and ExoY (Shaver et al., Infect. Immun., 72, 6969-6977 (2004)). ExoS has been the subject of particular interest. This bifunctional toxin possesses an N-terminal Rho GTPase activating protein (GAP) activity (Goehring et al., J. Biol. Chem., 274, 36369-36372 (1999); Wurtele et al., FEBS Lett, 491, 26-29 (2001); Henriksson et al., Biochemical Journal, 347, 217-222 (2000)) and a highly promiscuous C-terminally encoded ADP-ribosylation activity towards small GTP-binding proteins, such as the RAS family members (Henriksson et al., Biochem. J., 367, 617-628 (2002)). Activation of the ADP-ribosyltransferase activity of ExoS results in cell death (Liu et al., Biochemistry, 35, 2754-2758 (1996); Frithz-Lindsten et al., Mol. Microbiol., 25, 1125-1139 (1997)) (see Figure 1A) and, in animal models of pneumonia, increased bacterial persistence in the lung and decreased host survival (Yahr et al., Mol. Microbiol., 22, 991-1003 (1996)). In order to activate some of its pathogenic action ExoS need to form a complex with the host protein 14-3-3. A crystal structure of the 14-3-3 binding domain of ExoS and 14-3-3 was reported by Ottmann and coworkers (Ottmann et al., EMBO J., 26, 902-913 (2007)). In this structure ExoS is binding to 14-3-3 with the following residues: ⁴¹⁶SGHGQGLLDALDLAS⁴³⁰ (see Figure 1B). This crystal structure and an alanine scan revealed the importance of ExoS leucine residues at positions 422, 423, 426 and 428 for binding (Ottmann et al., EMBO J., 26, 902-913 (2007)).

Inhibition of protein-protein interactions (PPIs) by cell-permeable molecules is considered particularly challenging. Especially extended protein-protein interfaces without hydrophobic pockets are difficult to target by classical small molecule approaches. Interfaces of PPIs are defined by participating secondary structures. Therefore, such secondary structure elements were envisioned as dominant interfering peptides for the inhibition of protein-protein interactions (Verdine et al., Clin. Cancer Res., 13, 7264-7270 (2007)). However, such small peptide sequences usually exhibit little or no secondary structure when excised from the stabilizing protein context resulting in reduced affinity, cell permeability and proteolytic stability. Consequently, approaches that enforce secondary structure upon relatively short peptide sequences were developed for designing peptide-derived inhibitors of protein-protein interactions (Wilson, Chem. Soc. Rev., 38, 3289-3300 (2009)).

Stabilization of β-sheets was facilitated by macro-cyclization and introduction of β-turn mimetics (Robinson, Accounts Chem. Res., 41, 1278-1288 (2008)). For α-helices a number of stabilization approaches was reported, most of them involving the cross-linking of neighboring residues facing the same side of the helix (Henchey et al., Curr. Opin. Chem. Biol, 12, 692-697 (2008)). *'Peptide stapling'* is one such strategy that involves the introduction of a synthetic hydrocarbon bridge into an α-helical peptide by incorporating two α-methyl,α-alkenyl amino acids during chain extension in solid-phase peptide synthesis (Kim et al., Nat. Protocols, 6, 761-771 (2011)). This is followed by closure of the macrocyclic bridge using ruthenium-mediated ring-closing olefin metathesis (Kim et al., Nat. Protocols, 6, 761-771 (2011)). The peptide stapling technology proved to increase the binding affinity, to decrease the rate of proteolytic degradation and to support cell penetration of α-helical interaction motifs (Walensky et al., Science, 305, 1466-1470 (2004); Moellering et al., Nature, 462, 182-188 (2009); Grossmann et al., Proc. Natl. Acad. Sci. USA., 109, 17942-17947 (2012)). A good starting point for the design of stapled peptides is a structurally characterized α-helical interaction motif. In general, the first step involves the identification of appropriate sites for an incorporation of the non-natural amino acids used to form the cross-link. Typically, the art uses those residues that are not involved in target recognition to introduce different stapling architectures (*i*,*i*+3; *i*,*i*+4; or *i*,*i*+7) (Schafmeister et al., J. Am. Chem. Soc., 122, 5891-5892 (2000); Kim et al., Org. Lett., 12, 3046-3049 (2010)). The generated library of stapled peptides is then tested to ensure the identification of the optimal stapling scaffold.

WO 2005/044839 describes α-helical peptides which have been stabilized by means of hydrocarbon stapling. As regards the mechanism of action of described peptides, WO 2005/044839 refers to the triggering of apoptosis. Schafmeister et al. (*loc. cit.*) describes peptides which have been cross-linked by means of hydrocarbon stapling. The parent sequence has been taken from the amino acid sequence of RNAse A. Kim et al. (*loc. cit.*) describes a further development in the field of hydrocarbon stapling, namely the generation of (i, i+3) cross links. Grossmann et al. (*loc. cit.*) describes a therapeutic use of peptides which have been cross-linked by means of hydrocarbon stapling. The authors describe that a target which generally is being viewed as difficult (β-catenin) could be addressed with cross-linked α-helical peptides.

There are examples that describe the use of a peptide (containing only natural amino acids (Wang et al., Biochem., 38, 12499-12504 (1999); Petosa et al., J. Biol. Chem., 273, 16305 - 16310 (1998)) and of low molecular weight molecules (Corradi et al., Bioorg. Med. Chem., 20, 6133 - 6137 (2010); Wu et al., Angew. Chem. Int. Ed., 49, 6528-6532 (2010); Zhao et al., Proc. Natl. Acad. Sci. USA 108, 16212-16216 (2011); Arrendale et al., Chem. Biol., 19, 764-771 (2012)) as inhibitors of a PPI, wherein a 14-3-3 protein is participating in this interaction. The other interaction partners are endogenous phosphorylated substrates which play a role in the indications of cancer and neurodegenerative disorders.

In view of the prior art, to the extent it is pertinent, the technical problem underlying the present invention may be seen in the provision of alternative or improved means of treating *Pseudomonas* infections, in particular infections by multiply resistant *Pseudonomas aeruginosa* strains or health-care associated infections caused by *Pseudonomas aeruginosa.*

Accordingly, this invention provides in a first aspect a peptide or depsipeptide of formula (I)

(Y)ₖX¹YZ¹X²Z²(Y)ₘ (I),

wherein X¹ is an α-amino acid residue or an α-hydroxy acid residue of formula (IIa)

-A-CR¹R²-CO- (IIa),

X² is an α-amino acid residue or an α-hydroxy acid residue of formula (IIb)

-A-CR³R⁴-CO- (IIb),

wherein A is NH or O; R¹ and R³ are independently selected from H, halogen such as F, substituted or unsubstituted C₁ to C₄ alkyl such as methyl, substituted or unsubstituted aryl and substituted or unsubstituted arylalkyl, aryl being a 5- or 6-membered ring with 0, 1, 2 or 3 heteroatoms, heteroatoms being selected from O, N and S, alkyl in said arylalkyl being C₁ to C₄ alkyl, said alkyl in said arylalkyl optionally being substituted, the term "substituted", when relating to R¹ and R³, providing for, as valence permits, 1, 2, 3, 4, 5, 6, 7 or 8 substituents, said substituents being chosen from halogen such as F; R² and R⁴ together are chosen to be substituted or unsubstituted n-alk-1,ω-diyl or substituted or unsubstituted n-alken-1,ω-diyl, ω being an integer number selected from 6, 7, 8, 9, 10, 11, 12, 13 and 14, the term "substituted", when relating to R² and R⁴, providing for 1 or 2, in case of the substituents being halogen 1, 2, 3, 4, 5, 6, 7 or 8 substituents, said substituents being chosen from halogen such as F and C₁ to C₄ alkyl such as methyl, said C₁ to C₄ alkyl in turn optionally being substituted with, as valence permits, 1, 2, 3, 4, 5, 6, 7 or 8 substituents, said substituents being chosen from halogen such as F, and the double bond in said n-alken-1,ω-diyl being at any one of positions 1, 2, 3, 4,... and (ω-1); each occurrence of Y is independently selected to be an α-amino acid or α-hydroxy acid; k is an integer number selected from 0, 1, 2, 3, 4, 5, 6 and 7; m is an integer number selected from 1, 2, 3 and 4; Z¹ is a polar or negatively charged α-amino acid or α-hydroxy acid; and Z² is a an aliphatic hydrophobic α-amino acid or α-hydroxy acid; the N-terminus of said peptide or depsipeptide is free; protected, preferably acetylated; or conjugated, preferably to a cell-penetrating peptide or a lipid; and the C-terminus of said peptide or depsipeptide is free; protected, preferably amidated; or conjugated, preferably to a cell-penetrating peptide or a lipid.

The terms "peptide" and "depsipeptide" have their art-established meaning. In particular, a peptide is a polycondensate of about 30 or less amino acids. A depsipeptide may be viewed as a derivative of a peptide, wherein one or more peptide bonds between adjacent amino acids have been replaced with ester bonds. The residue located at the C-terminal end of the ester bond provides a hydroxy group for the formation of the ester bond. As a consequence, said C-terminal residue of an ester bond, in its monomeric form, is an α-hydroxy acid. Preference is given to peptides and depsipeptides with a low proportion of ester bonds such as 1, 2, 3, 4 or 5 ester bonds. As noted further below, to the extent α-amino acids are the building blocks, preference is given to 20 proteinogenic amino acids. Similarly, to the extent depsipeptides are envisaged, the α-hydroxy acid residues thereof are preferably those α-hydroxy acids which are the counterparts of the 20 proteinogenic α-amino acids. To give an example, lactic acid is an α-hydroxy acid and may be viewed as the hydroxy analogue of alanine. The same considerations apply *mutatis mutandis* to the remainder of the proteinogenic amino acids, the α-hydroxy counterparts thereof being envisaged as monomers of depsipeptides in accordance with the present invention.

As is apparent from formula (I), peptides or depsipeptides in accordance with the present invention typically have a minimal length of 6 (in this case k would be 0 and m would be 1) and a maximal length of 16 (k in that case being 7 and m being 4).

The core part of the peptide or depsipeptide sequence is defined by the pentameric sequence X¹YZ¹X²Z². The five residues constituting this core sequence are defined above. In particular, Z¹ is a polar or negatively charged α-amino acid or a polar or negatively charged α-hydroxy acid. Preferred negatively charged amino acids are Asp and Glu. Their α-hydroxy acid counterparts are α-hydroxy butanedioic acid and α-hydroxy pentanedioic acid, respectively. Polar α-amino acids include Ser, Thr, Asn and Gln. Again, to the extent the invention extends to depsipeptides, the α-hydroxy acid counterparts of these four polar amino acids are preferred.

Preferred aliphatic hydrophobic α-amino acids to be used in position Z² are Ala, Val, Ile, Leu and Met. The α-hydroxy acid counterparts of these five aliphatic hydrophobic amino acids are preferred building blocks of depsipeptides in accordance with the present invention.

Y is a variable position, wherein preference is given to one of the proteinogenic α-amino acids.

X¹ and X² are special positions in that they define the end points of the hydrocarbon cross-link in accordance with the present invention, said hydrocarbon cross-link being represented by moieties R² and R⁴ together. In particular, R² and R⁴ together are to be chosen as defined herein above, wherein preference is given to unsubstituted n-alk-1,ω-diyl. The prefix "n-" in "n-alk-1,ω-diyl" etc. has the art-established meaning and refers to unbranched alkane and alkene moieties. It is not to be confused with any subscript or index as it might be used herein.

While it is stated above that preference is given to the proteinogenic amino acids, this does not mean that (i) the R-counterparts of the proteinogenic amino acids (which as such have S stereochemistry at the α-carbon; only Gly is non-chiral) would be excluded. Yet further, while the proteinogenic amino acids are characterized in that the α-carbon carries a side chain and a hydrogen, also the use of α-amino acids is envisaged which have two substituents at the α-carbon.

Preference is given to unsubstituted R¹ and R³.

Preference is given to unsubstituted R² and R⁴

Preferred C₁ to C₄ alkyl moieties to be used as moieties R¹ and R³, and furthermore as substituents in R² and R⁴ are methyl, ethyl, n-propyl and isopropyl, each one of which may be halogenated as defined above. Particularly preferred are methyl and trifluoromethyl. As noted above, R² and R⁴ together define the cross-link which cross-link as a whole may be substituted, e.g. with up to two C₁ to C₄ alkyl moieties and/or up to 8 halogen atoms.

A preferred aryl moiety is phenyl. A preferred aryl alkyl moiety is benzyl.

The term "halogen" refers to F, Cl, Br, and I, wherein preference is given to F. The latter does not exclude that in those cases where two or more halogen substituents are present, said two or more halogen substituents differ from each other.

Preferred values of ω are 8, 9, 10, 11 and 12; 8, 10, 11 and 12 being especially preferred.

Preference is given to R² and R⁴ together being unsubstituted n-alk-1,ω-diyl. It is noted that, without being bound by a particular theory, it might be beneficial to further enhance the hydrophobicity of the moiety formed by R² and R⁴ together. To explain further, and contrary to prior art approaches, the cross-link in accordance with the present invention is chosen such that it makes contact to the target protein (which is a 14-3-3 protein in the present case). Therefore, the introduction of a double bond and/or of hydrophobic substituents (halogen, lower alkyl) in the linker is envisaged.

As noted above, N- and/or C-terminus may be protected. Suitable protection groups for the N-terminus include acetyl and CH₃-(CH₂)ₗ-CO-, I being an integer number from 1 to 18. Suitable protection groups for the C-terminus include -NH-(CH₂)ₚ-NH₂ with p being 2, 3, 4, 5 or 6 and -NH-(CH₂)ₚ-NR'₃⁺ with p being 2, 3, 4, 5 or 6 and R' being C₁ to C₄ alkyl such as methyl. Furthermore, one or both termini may be conjugated to lipids. Preferred lipids include all naturally occuring fatty acids including palmitic acid, stearic acid, arachidic acid, oleic acid, acharidonic acid, linoleic acid, and linolenic acid. Also, a cell penetrating peptide may be conjugated to one (or both) of the two termini. It is noted that in the latter case the total number of amino acids may exceed 16 as specified above. Further information on cell penetrating peptides is at the skilled person's disposal; as an example we refer to Gautam et al. (CPPsite: a curated database of cell penetrating peptides; Database (Oxford). 2012 Mar. 7;2012:bas015. doi: 10.1093/databse/bas015.) which reference describes a curated publicly available database of cell penetrating peptides. Preferred CPPs are oligo arginine-sequences such as (Arg)q, q being an integer number from 3 to 9.

The first aspect of the invention provides peptides and depsipeptides which are derived from a subsequence of the *Pseudonomoas aeruginosa* protein ExoS. The examples enclosed herewith illustrate the present inventors' approach of identifying a subsequence of the ExoS protein which subsequence in turn serves as a basis for the design of the peptide and depsipeptide according to the present invention which is cross-linked. The peptides and depsipeptides of the invention decrease binding affinity of the *P. aeruginosa* ExoS protein to a 14-3-3 protein, preferably a human 14-3-3 protein, or prevent binding of ExoS to 14-3-3 altogether. This principle is illustrated in Figure 2 as enclosed herein. It is apparent from Figure 2B that the binding sites for ExoS on the one hand and endogenous 14-3-3 binders on the other hand, while being adjacent, overlap only to some extent. While this is not required, preference is given to peptides and depsipeptides of the present invention which do not interfere with the binding of endogenous 14-3-3 binders to a 14-3-3 protein.

The hydrocarbon cross-links can enhance proteolytic stability and cell permeability of the peptides and depsipeptides. As regards the placement of the hydrocarbon cross-link within the peptide or depsipeptide, the present inventors have chosen an unconventional approach which surprisingly turned out to be beneficial. In particular, the cross-link is placed in the 14-3-3 binding interface of the peptide. While the prior art uses hydrocarbon staples to stabilize secondary structure of a given peptide, the known approaches deliberately place the hydrocarbon staple such that it is not expected to make contacts with the target molecule. Related to the present inventors' unconventional approach, it is furthermore disclosed herein that the cross-link may be derivatized further such that hydrophobic interactions between the peptide or depsipeptide according to the present invention on the one hand and a 14-3-3 protein on the other hand are reinforced.

A yet further surprising effect is that the peptides and depsipeptides according to the present invention, when bound to the 14-3-3 protein, are not α-helical throughout. This amounts to a significant departure from the prior art which uses hydrocarbon stapling generally to stabilize and maintain an α-helical structure.

Since the peptides and depsipeptides according to the invention interfere with the binding of ExoS to a 14-3-3 protein, they provide a novel antibiotic approach in the treatment of *P*. *aeruginosa* infections.

In a preferred embodiment, the stereochemistry of the α-carbons of X¹ and X² is R and S, or S and S, respectively. The former scenario is briefly referred to as "RS", and the latter as "SS". RS stereochemistry is being especially preferred in conjunction with shorter cross links, in particular cross links with 8 carbon atoms. The SS stereochemistry is especially preferred in conjunction with longer cross links such as cross links consisting of 10, 11 and 12 carbon atoms. Both the SS stereochemistry as well as the length of 10, 11 and 12 carbon atoms are surprisingly found to provide for active peptides or depsipeptides. This is not suggested in the art.

In a further preferred embodiment, R³ is methyl. In conjunction therewith, R¹ may be freely chosen, preferably to be H.

Yet, in a further preferred embodiment, R¹ is methyl.

In a further preferred embodiment, (a) Y between X¹ and Z¹ is Leu; (b) Z¹ is Asp or Asn; and/or (c) Z² is Leu.

In a further preferred embodiment, (a) k is 0, 1 or 2; and/or (b) m is 2, 3 or 4.

In a further preferred embodiment (a) (Y)ₙ is Gln-Gly or Ala-Gly; and/or (b) (Y)ₘ is selected from Asp-Leu-Ala-Ser, Asn-Leu-Ala-Ser, Asp-Leu-Ala, Asn-Leu-Ala, Asp-Leu and Asn-Leu.

In a further preferred embodiment one, two or three occurrences of Y are Arg. This embodiment provides for the enhancement of the cell penetrating properties of the peptides and depsipeptides according to the present invention. The presence of one, two or three arginines may be used with or without a cell penetrating peptide being conjugated to the peptide or depsipeptide according to the present invention.

We note that in the parent sequence as shown in Table 1 further below, arginines are absent. Therefore, and in an alternate preferred embodiment, the sequence of the peptide or depsipeptide according to the present invention may be designed in closer or full adherence to the sequence of the parent ExoS subsequence as shown in the third line of Table 1.

In a further preferred embodiment, said unsubstituted alk-1,ω-diyl is (a) n-oct-1,8-diyl, (b) n-dec-1,10-diyl, (c) n-undec-1,11-diyl, or (d) n-dodec-1,12-diyl.

In conjunction with option (a) of the latter embodiment, it is preferred that the stereochemistry of the α-carbons of X¹ and X² is R and S, respectively.

On the other hand and in conjunction with options (b) to (d), it is preferred that the stereochemistry of the α-carbons of X¹ and X² is S and S, respectively.

In a further preferred embodiment and as noted herein above, all α-amino acids are selected from the 20 proteinogenic α-amino acids. Said proteinogenic α-amino acids are well known and are the following: Ala, Arg, Asn, Asp, Cys, Glu, Gln, Gly, His, Ile, Leu, Lys, Met, Phe, Pro, Ser, Thr, Trp, Tyr and Val.

In a further preferred embodiment, said peptide binds to one or more 14-3-3 proteins, the 14-3-3 protein preferably being selected from the proteins consisting of the amino acid sequence of any one of SEQ ID NO: 1 to 7.

In a further preferred embodiment, said peptide, when bound to said 14-3-3 protein, makes contacts with one, more or all specific residues of said 14-3-3 protein, said specific residues of said 14-3-3 protein being selected from N42, S45, V46, K49, F117 and D213 of the 14-3-3 protein of SEQ ID NO: 4 or residues of any one of SEQ ID NOs: 1 to 3 or 5 to 7 aligning with residues N42, S45, V46, K49, F117 and D213 of SEQ ID NO: 4.

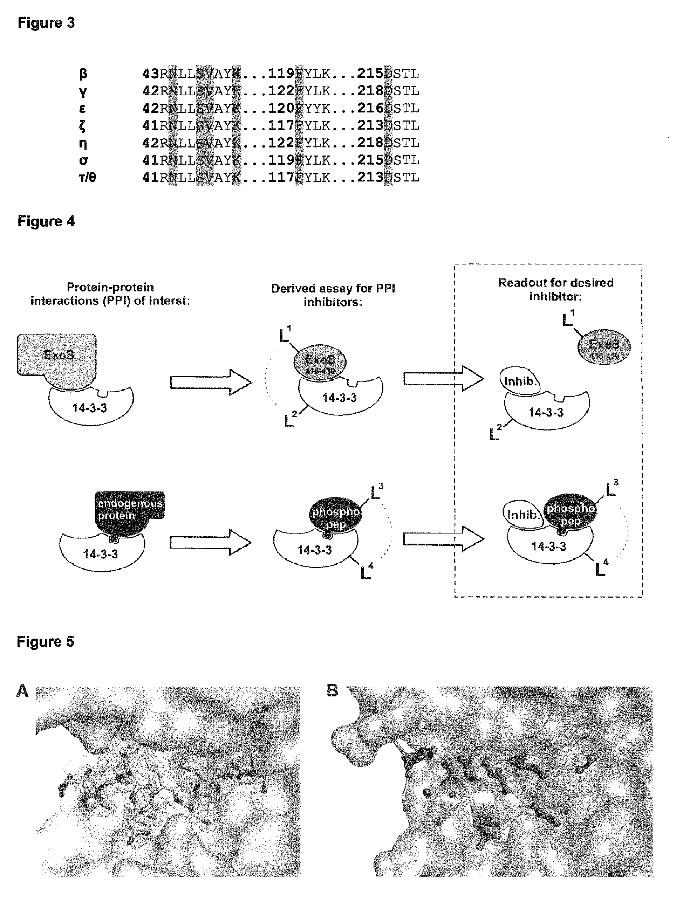

The residues of the human 14-3-3 proteins having the sequence of SEQ ID NOs: 1 to 7, said residues making contact with the peptides and depsipeptides according to the present invention, are strictly conserved; see Figure 3 enclosed herewith. Figure 3 shows residues of any one of SEQ ID NOs: 1 to 3 or 5 to 7 aligning with residues N42, S45, V46, K49, F117 and D213 of SEQ ID NO: 4.

In a particularly preferred embodiment, at least one, at least two or at least three of said contacts involve said substituted or unsubstituted n-alk-1,ω-diyl or said substituted or unsubstituted n-alken-1,ω-diyl.

In a further preferred embodiment, said peptide is a peptide of formula (III) or (IV)

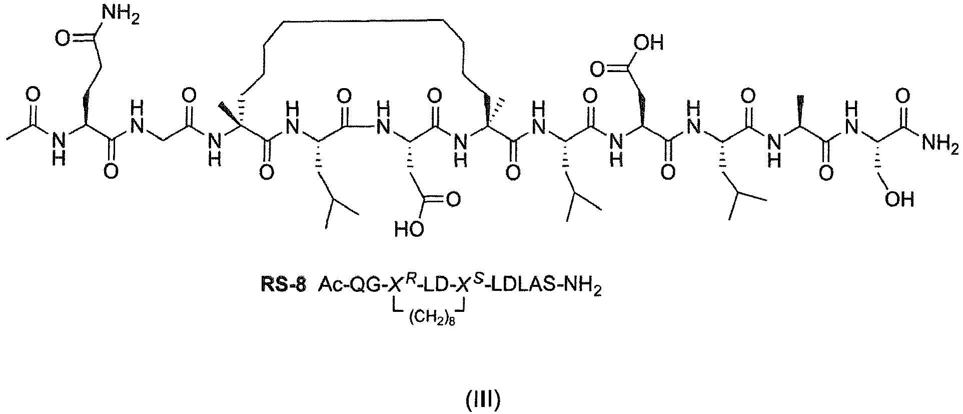

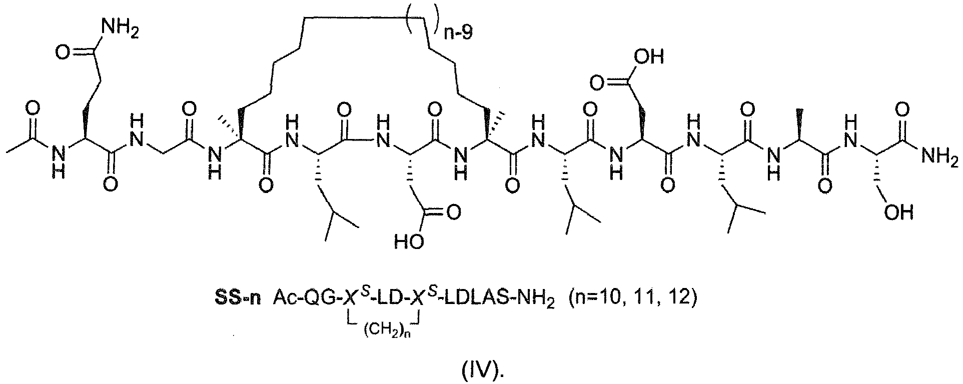

In formulae (III) and (IV) both the atomic structure and a simplified representation of the peptide using the single letter code for amino acids is shown. In either case, the positions X¹ and X² in accordance with formula (I) are indicated with their respective stereochemistry (X^{R} and X^{S}). Furthermore, the designation system used by the present inventors ("RS-8" and "SS-n", n being 10, 11 or 12 in the context of formula (IV)) is shown, wherein the designation system captures the key features of the cross-link: (i) the stereochemistry of the α-carbons in amino acids X¹ and X², and (ii) the number of carbon atoms in the cross-link. It is noted that in the particularly preferred compounds in accordance with formulae (III) and (IV), both R¹ and R³ are methyl.

In a second aspect, the present invention provides a pharmaceutical composition comprising a peptide or depsipeptide in accordance with the present invention.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the peptide or depsipeptide. For example, the total pharmaceutically effective amount of pharmaceutical composition administered parenterally per dose will be in the range of about 1 µg peptide or depsipeptide /kg/day to 10 mg peptide or depsipeptide /kg/day of patient body weight, although, as noted above, this will be subject to therapeutic discretion. More preferably, this dose is at least 0.01 mg peptide or depsipeptide /kg/day, and most preferably for humans between about 0.01 and 1 mg peptide or depsipeptide /kg/day. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers, 22, 547-556 (1983)), poly (2-hydroxyethyl methacrylate) (R. Langer et al., J. Biomed. Mater. Res., 15, 167-277 (1981), and R. Langer, Chem. Tech., 12, 98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. USA, 82, 3688-3692 (1985); Hwang et al., Proc. Natl. Acad. Sci. USA, 77, 4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle. The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The solution for infusion or injection is prepared by reconstituting the lyophilized compound(s) using bacteriostatic Water-for-Injection.

In a third aspect, the present invention provides a peptide or depsipeptide according to the present invention for use in a method of treating or preventing a *Pseudomonas aeruginosa* infection.

In a fourth aspect, the present invention provides a method of treating or preventing a *Pseudomonas aeruginosa* infection in a subject, said method comprising administering to said subject a therapeutically active amount or a preventive amount, respectively, of a peptide or depsipeptide in accordance with the present invention.

Suitable therapeutically or preventive amounts are described herein above in relation to the pharmaceutical composition in accordance with the second aspect.

In a preferred embodiment of the third and fourth aspect, said *Pseudomonas aeruginosa* infection is (a) an infection by a *Pseudomonas aeruginosa* strain which is resistant to one or more antibiotics, said antibiotics preferably being selected from Cefepime, Ceftazidime, Aztreonam, Piperacillin-tazobactam, Imipenem, Meropenem, Ciprofloxacin, Levofloxacin, Amikacin, Tobramycin and Gentamicin; (b) a hospital-acquired infection; and/or (c) affecting a subject (i) suffering from cystic fibrosis; (ii) suffering from an HIV infection; and/or (iii) which is immune suppressed.

In a fifth aspect, the present invention provides the use of a peptide or depsipeptide of the invention as a lead compound for the development of a drug, said drug being for treating or preventing a *Pseudomonas aeruginosa* infection in a subject.

In a preferred embodiment of the fifth aspect, said development, in its preclinical stage, involves the testing of a peptide or depsipeptide of the invention (a) in a cellular assay using cells infected with *Pseudomonas aeruginosa,* said cells preferably being HeLa or HEK293 cells; and/or (b) in a mouse suffering from pneumonia caused by *Pseudomonas aeruginosa.*

Said mouse is preferably chosen to be a standard laboratory strain such as BALB/c.

In a sixth aspect, the present invention provides an *in vitro* method of eliminating or preventing a *Pseudomonas aeruginosa* infection of a cell in culture, said method comprising bringing into contact said cell with a peptide or depsipeptide according to the invention.

In a seventh aspect, the present invention provides a method for identifying a compound (Figure 4), wherein said method comprises (a) (aa) bringing into contact *Pseudomonas aeruginosa* ExoS protein or a fragment thereof, said fragment comprising the residues of said ExoS protein binding to a 14-3-3 protein, with said 14-3-3 protein, wherein said ExoS protein or fragment thereof comprises a label L¹ and said 14-3-3 protein comprises a label L²; (ab) determining fluorescence of said label(s) L¹ and/or L² prior to and after said bringing into contact; wherein a change in said fluorescence of said label(s) L¹ and/or L² is indicative of a compound which interferes with the binding of said *Pseudomonas aeruginosa* ExoS protein to said 14-3-3 protein; (b) (ba) bringing into contact said 14-3-3 protein and a cognate endogenous phosphorylated protein or fragment thereof, said fragment comprising the residues of said phosphorylated protein binding to said 14-3-3 protein, wherein said phosphorylated protein or fragment thereof comprises a label L³ and said 14-3-3 protein comprises a label L⁴; (bb) determining fluorescence of said label(s) L³ and/or L⁴ prior to and after said bringing into contact; wherein no change in said fluorescence of said label(s) L³ and/or L⁴ is indicative of said compound not interfering with the binding of a cognate endogenous phosphorylated protein to said 14-3-3 protein.

This aspect relates to a screening method, wherein part (a) of the screening method ensures that the compound to be identified interferes with the binding of ExoS to 14-3-3 and part (b) of the screening method ensures that binding of the compound to be identified does not or not significantly interfere with binding of endogenous phosphorylated substrate to 14-3-3. The principle is illustrated in Figure 4 enclosed herewith.

The figures show:
**Figure 1****:** (A) Schemetic representaion of ExoS' role in P. aeruginosa infection. (B) Crystal structure of 14-3-3 binding domain of ExoS bound to 14-3-3 (PDB 2002).
**Figure 2****:** (A) Concept for an antibiotic strategy against *P. aeruginosa.* (B) Overlaid structures of 14-3-3 in complex with 14-3-3 binding domains of ExoS (PDB 2002) and endogenous phosphorylated sequences (PDB 1QJA, 1YWT, 2B05, 2BR9, 2BTP, 2C1J, 2C1N, 2C63, 2C74, 2NPM, 2IQV, 3LW1).
**Figure 3****:** Alignment of 14-3-3 residues involved in inhibitor binding.
**Figure 4****:** Dual assay for the identification of inhibitors of 14-3-3/ExoS interaction that would not affect interactions of 14-3-3 with endogenous binding parterns. L¹ and L² as well as L³ and L⁴ are chosen in order to allow a fluorescence polarization readout, ALPHA screen ("Amplified Luminescent Proximity Homogeneous Assay") or HRTF screen ("homogeneous time resolved fluorescence"; both ALPHA and HRTF screens are very sensitive homogeneous assay technologies that allow the screening of interactions in a proximity sensitive fashion).
**Figure 5****:** (A) Crystal structure of ⁴²⁰QGLLDALDLAS⁴³⁰ (sticks) complexed to 14-3-3 ζ ΔC (spacefill). The 2*F_{C} - F₀* density is scaled to 1σ (grid). (B) Superimposition of truncated ExoS peptide (⁴²⁰QGLLDALDLAS⁴³⁰) with the previously reported ExoS peptide (⁴¹⁶SGHSQGLLDALDLAS⁴³⁰, PDB 2002). Both peptides bind to 14-3-3 ζ in almost identical manner. The ionic and hydrophobic interactions allow phosphorylation-independent binding of the peptide to 14-3-3.
**Figure 6****:** (A) Schematic representation of different cross-link architectures. (B) FP measurements of three different cross-link architectures with corresponding dissociation constants (K_{D}). (C) K_{D}-values relative to WT peptide for β cross-links only (X^{R}X^{S}: absolute configuration of α-carbons in cross-link - first N- (R) then C-terminal (S) α-carbon, X^{S}X^{S}: absolute configuration of α-carbons in cross-link - first N- (S) then C-terminal (S) α-carbon, linker length: number of C-atoms in the crosslink).
**Figure 7****:** FP competition experiment with FITC-labeled WT (ExoS⁴²⁰⁻⁴³⁰) or RS-8 (20 nM) competing with unlabeled WT for 14-3-3 (4.0 µM) binding (FITC: fluorescein isothiocyanate).
**Figure 8****:** (A) Front and 90° tilted view of 14-3-3 ζ ΔC (ribbons). Protein is complexed to RS-8 peptide (sticks). The cross-link is highlighted as red ball and stick representation. (B) Crystal structure of RS-8 peptide (sticks) complexed to 14-3-3 ζ ΔC (spacefill). The 2*F_{C}* - *F₀* density is scaled to 1σ (grid). The enlarged view shows distinct density of the cross-link between amino acids in positions 422 and 425.
**Figure 9****:** (A) Schematic representation of interactions between ExoS and 14-3-3 calculated using the program LigPlot. (B) and (C) Front and side view of overlaid structure of wild type peptide (black, PDB 2002) and RS-8 peptide (grey) in complex with 14-3-3.

The examples illustrate the invention.

### Example 1

### Investigation of 14-3-3 binding domain of ExoS

Previously, the 14-3-3 binding domain of ExoS was structurally characterized by Ottmann and coworkers (Ottmann et al., Embo J., 26, 902-913 (2007)). Their crystal structure showed residues 416 - 430 of ExoS bound to 14-3-3. At first, we decided to investigate if shorter versions of the 14-3-3 binding domain of ExoS would exhibit sufficient binding to 14-3-3. Therefore, different ExoS derived peptides were synthesized via Fmoc-based solid phase peptide synthesis (SPPS) using the above mentioned 15-mer peptide (⁴¹⁶SGHSQGLLDALDLAS⁴³⁰) as starting point. A fluorescence polarization (FP) assay was used to determine the affinity of fluorescein-labeled truncated versions of this peptide to 14-3-3 (Table 1). The peptides were modified with a poly ethylene glycol (PEG) spacer and labeled with fluorescein isothiocyanate. The labeled peptides were titrated with unlabeled 14-3-3 protein. The shorter versions showed similar but slightly reduced affinities compared to the 15-mer starting sequence (Table 1).

**Table 1: Peptides derived from the 14-3-3 binding domain of ExoS and their dissociation constant (K_{D}) with 14-3-3 obtained from FP measurements.**

| **Sequence** | **K_{D} [µM]** |
|---|---|
| ⁴¹⁶SGHSQGLLDALDLAS⁴³⁰ | 0.61 |
| ⁴¹⁸HSQGLLDALDLAS⁴³⁰ | 0.72 |
| ⁴²⁰QGLLDALDLAS⁴³⁰ | 1.1 |

We decided to proceed with the 11-mer (⁴²⁰QGLLDALDLAS⁴³⁰). To confirm its binding mode the synthetic ExoS binding sequence was co-crystallized with the 14-3-3 isoform ζ. The crystallographic trials were performed with a truncated version of the 14-3-3 ζ ΔC (ΔC = amino acids 1-230). This Protein was expressed in *E. coli* and purified using standard purification techniques. The complex, used in the crystallization screen (Qiagen, NeXtal Screens), was prepared in a molar ratio of 2:1 (14-3-3/ExoS peptide). The protein-peptide complex led to crystals after three weeks in the following buffer condition: 0.2 M Sodium chloride, 0.1 M sodium acetate pH 4.5 and 40% (v/v) PEG 300, and showed diffraction to 1.9 Å. After molecular replacement, the space group was determined to be P 3₁ 2 1. The amphipathic binding groove of 14-3-3 ζ showed a clearly defined density for the entire peptide, allowing the incorporation of the whole sequence (Figure 5A). The peptide binds in a phosphorylation independent manner in the binding groove of 14-3-3 ζ. In most cases the arrangement of amino acids Lys49, Arg56, Arg127 and Tyr128 are responsible for the coordination of a phosphorylated serine or threonine (mode I-III). In our case, this position remains unoccupied. The superimposition of our structure with the previously reported structure involving the longer ExoS version (PDB 2002) (Ottmann et al., EMBO J., 26, 902-913 (2007)) showed that both ExoS sequences overlay almost perfectly (Figure 5B). This also involves the formation of the short α-helical structure comprising the hydrophobic residues Leu422, Leu423, Leu426 and Leu428 that bind to a hydrophobic area in the amphipathic groove of 14-3-3.

### Example 2

### Investigation of different cross-link architectures

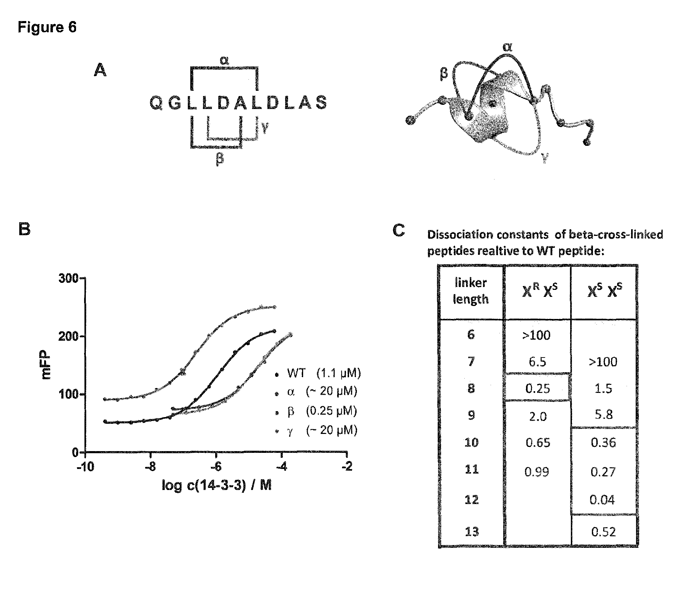

To stabilize the secondary structure we decided to use the peptide stapling approach. Therefore, suitable amino acids were replaced by unnatural olefinic amino acids (usually 2-amino-2-methyl-hept-6-enoic acid, called S5 for the S- and R5 for the R-enantiomer). Subsequently, the hydrocarbon cross-link was formed using ring closing metathesis. For the stabilization three different cross-link positions were chosen and synthesized via SPPS (Figure 6A). For the α cross-link (*i*,*i*+4) the residues Leu426 and Leu422 were replaced with S5. The residue Ala425 was replaced with R5 and Leu422 with S5 for the β cross-link (*i*,*i*+3). And the γ cross-link (*i*,*i*+3) employs positions Leu426 and Leu423 (R5 and S5, respectively). The ring closing metathesis was performed with Grubbs catalyst I and the double bonds were reduced with 2,4,6,-Triisopropylbenzenesulfonyl hydrazide. The binding affinities were again determined using FP. The obtained binding curves with 14-3-3 and corresponding dissociation constants (K_{D}) are shown in Figure 6B. Compared to the starting sequence (WT) only the β cross-linked peptide shows increased affinity for 14-3-3 while the α and the γ cross-linked peptides exhibit a significantly decreased binding affinity (Figure 6B). As a consequence only β cross-linked peptides are considered further on.

In order to investigate the influence of the length of the cross-link of the α-carbon this parameter was varied (Figure 6C). Notably, the classic peptide stapling approach covers the initially tested versions of the α, β and γ cross-link (C8-linker). The binding affinity of the non-classic cross-linked peptides was investigated using FP (Figure 6C). Surprisingly, in addition to classic β cross-linked RS-8, some of the novel peptides showed relatively high binding affinities (e.g. SS-10, SS-11 and SS-12). These peptides provide a good starting point for further optimizations.

### Example 3

### Investigation of modified peptide RS-8

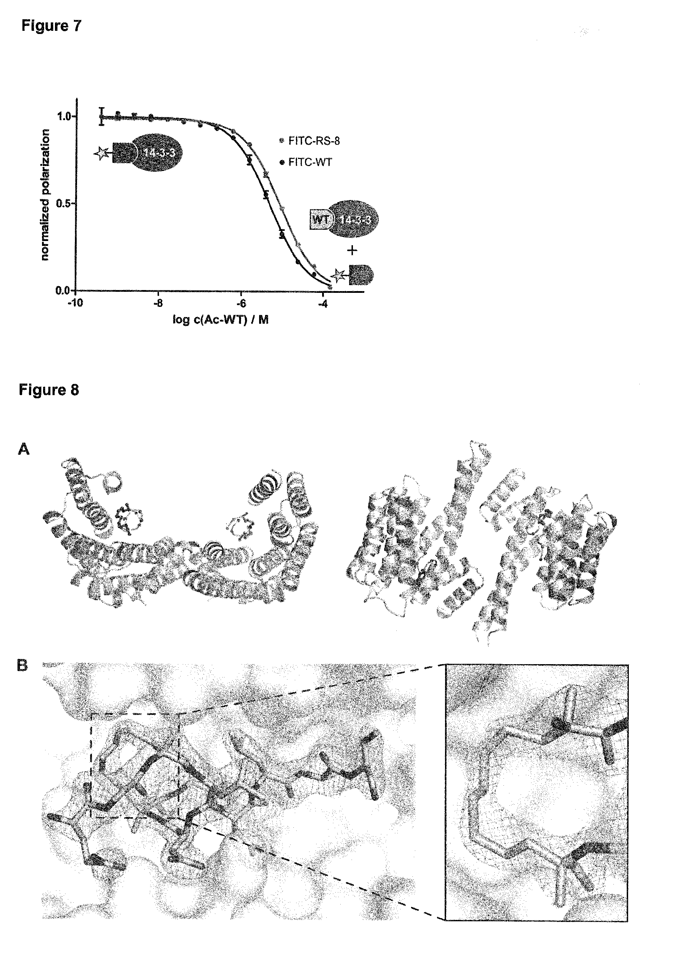

For further investigations we chose peptide RS-8. To confirm the binding site of RS-8 FP-competition measurements were performed. In this experiment, the labeled peptide was preincubated with 14-3-3 and titrated with unlabeled WT peptide (Figure 7). In case both peptides compete for the same binding site one should observe the displacement of the labeled peptide at high concentrations of the unlabeled WT peptide. In fact, we observe for both the labeled WT and RS-8 peptide a displacement by unlabeled WT peptide (Figure 7).

In order to determine the exact binding mode of RS-8 to 14-3-3 both were co-crystallized. The preparation of the complex and subsequent crystallographic trials have been carried out as described above. We used the same truncated 14-3-3 isoform ζ. The crystals grew within four weeks in the buffer condition: 0.09 M HEPES sodium salt pH 7.5, 1.26 M sodium citrate, 10 % (v/v) glycerol and scattered to a resolution of 2.1 Å. The 14-3-3 protein crystallizes in the P2₁2₁2₁ space group and shows typical W-like shape (Figure 8A). The density of the entire peptide including the introduced cross-link is clearly visible in both amphipathic grooves of 14-3-3 ζ (Figure 8B).

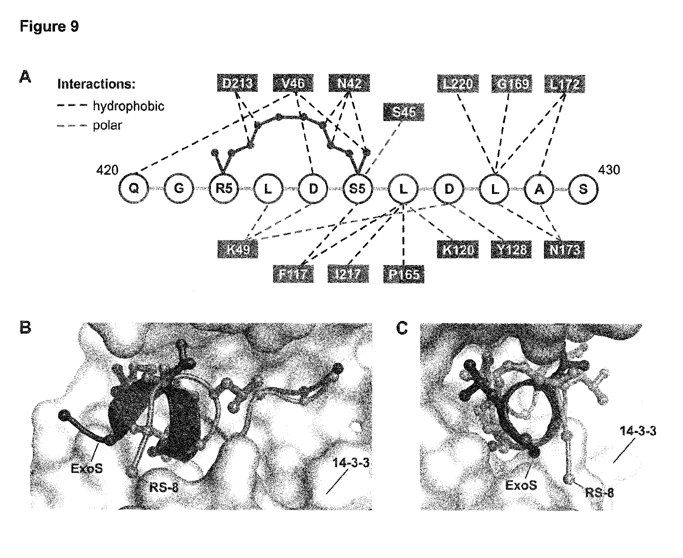

Similar to the WT peptide the C-terminal part of RS-8 forms ionic and hydrophobic interactions with 14-3-3 ζ (Figure 9A). Surprisingly, the N-terminal part of RS-8 adopts a completely different configuration than the WT peptide (Figure 9B and C). Leu426 and Leu428 are still directed into the hydrophobic region of the 14-3-3 protein (not shown) but Leu423 is shifted out of the binding groove (by approximately 5 Å) and does not contribute to intense hydrophobic interactions anymore. An explanation for this drastic rearrangement is the intense hydrophobic interaction of the β cross-link compensating for the loss of the shifted Leu423 interaction. This shift changes the conformation of the peptide backbone dramatically and prevents the formation of the expected α-helix (Figure 9B and C).

## Claims

1. A peptide or depsipeptide of formula (I)
(Y)ₖX¹YZ¹X²Z²(Y)ₘ (I),
wherein
X¹ is an α-amino acid residue or an α-hydroxy acid residue of formula (IIa)
-A-CR¹R²-CO- (IIa),
X² is an α-amino acid residue or an α-hydroxy acid residue of formula (IIb)
-A-CR³R⁴-CO- (IIb),
wherein
A is NH or O;
R¹ and R³ are independently selected from H, halogen such as F, substituted or unsubstituted C₁ to C₄ alkyl such as methyl, substituted or unsubstituted aryl and substituted or unsubstituted arylalkyl, aryl being a 5- or 6-membered ring with 0, 1, 2 or 3 heteroatoms, heteroatoms being selected from O, N and S, alkyl in said arylalkyl being C₁ to C₄ alkyl, said alkyl in said arylalkyl optionally being substituted, the term "substituted", when relating to R¹ and R³, providing for, as valence permits, 1, 2, 3, 4, 5, 6, 7 or 8 substituents, said substituents being chosen from halogen such as F;
R² and R⁴ together are chosen to be substituted or unsubstituted n-alk-1,ω-diyl or substituted or unsubstituted n-alken-1,ω-diyl, ω being an integer number selected from 6, 7, 8, 9, 10, 11, 12, 13 and 14, the term "substituted", when relating to R² and R⁴, providing for 1 or 2, in case of the substituents being halogen 1, 2, 3, 4, 5, 6, 7 or 8 substituents, said substituents being chosen from halogen such as F and C₁ to C₄ alkyl such as methyl, said C₁ to C₄ alkyl in turn optionally being substituted with, as valence permits, 1, 2, 3, 4, 5, 6, 7 or 8 substituents, said substituents being chosen from halogen such as F, and the double bond in said n-alken-1,ω-diyl being at any one of positions 1, 2, 3, 4,... and (ω-1);
each occurrence of Y is independently selected to be an α-amino acid or α-hydroxy acid;
k is an integer number selected from 0, 1, 2, 3, 4, 5, 6 and 7;
m is an integer number selected from 1, 2, 3 and 4;
Z¹ is a polar or negatively charged α-amino acid or α-hydroxy acid; and
Z² is a an aliphatic hydrophobic α-amino acid or α-hydroxy acid;
the N-terminus of said peptide or depsipeptide is free; protected, preferably acetylated; or conjugated, preferably to a cell-penetrating peptide or a lipid; and the C-terminus of said peptide or depsipeptide is free; protected, preferably amidated; or conjugated, preferably to a cell-penetrating peptide or a lipid.

2. The peptide or depsipeptide of claim 1, wherein
(a) Y between X¹ and Z¹ is Leu;
(b) Z¹ is Asp or Asn; and/or
(c) Z² is Leu.

3. The peptide or depsipeptide of claim 1 or 2, wherein
(a) (Y)ₖ is Gln-Gly or Ala-Gly; and/or
(b) (Y)ₘ is selected from Asp-Leu-Ala-Ser, Asn-Leu-Ala-Ser, Asp-Leu-Ala, Asn-Leu-Ala, Asp-Leu and Asn-Leu.

4. The peptide or depsipeptide of any one of claims 1 to 3, wherein one, two or three occurrences of Y are Arg.

5. The peptide or depsipeptide of any one of claims 1 to 4, wherein said unsubstituted alk-1,ω-diyl is
(a) n-oct-1,8-diyl,
(b) n-dec-1,10-diyl,
(c) n-undec-1,11-diyl, or
(d) n-dodec-1,12-diyl.

6. The peptide or depsipeptide
(a) of claim 5(a), wherein the stereochemistry of the α-carbons of X¹ and X² is R and S, respectively; or
(b) of any one of claims 5(b) to (d), wherein the stereochemistry of the α-carbons of X¹ and X² is S and S, respectively.

7. The peptide or depsipeptide of any one of the preceding claims, wherein all α-amino acids are selected from the 20 proteinogenic α-amino acids.

8. The peptide or depsipeptide of any one of the preceding claims, wherein said peptide binds to one or more 14-3-3 proteins, the 14-3-3 protein preferably being selected from the proteins consisting of the amino acid sequence of any one of SEQ ID NO: 1 to 7.

9. The peptide or depsipeptide of claim 8, wherein said peptide, when bound to said 14-3-3 protein, makes contacts with one, more or all specific residues of said 14-3-3 protein, said specific residues of said 14-3-3 protein being selected from N42, S45, V46, K49, F117 and D213 of the 14-3-3 protein of SEQ ID NO: 4 or residues of any one of SEQ ID NOs: 1 to 3 or 5 to 7 aligning with residues N42, S45, V46, K49, F117 and D213 of SEQ ID NO: 4.

10. The peptide or depsipeptide of claim 9, wherein at least one, at least two or at least three of said contacts involve said substituted or unsubstituted alk-1,ω-diyl or said substituted or unsubstituted alken-1,ω-diyl.

11. A pharmaceutical composition comprising a peptide or depsipeptide of any one of the preceding claims.

12. A peptide or depsipeptide of any one of claims 1 to 10 for use in a method of treating or preventing a *Pseudomonas aeruginosa* infection, wherein preferably said *Pseudomonas aeruginosa* infection is
(a) an infection by a *Pseudomonas aeruginosa* strain which is resistant to one or more antibiotics, said antibiotics preferably being selected from Cefepime, Ceftazidime, Aztreonam, Piperacillin-tazobactam, Imipenem, Meropenem, Ciprofloxacin, Levofloxacin, Amikacin, Tobramycin and Gentamicin;
(b) a hospital-acquired infection; and/or
(c) affecting a subject
(i) suffering from cystic fibrosis;
(ii) suffering from an HIV infection; and/or
(iii) which is immune suppressed.

13. Use of a peptide or depsipeptide of any one of claims 1 to 10 as a lead compound for the development of a drug, said drug being for treating or preventing a *Pseudomonas aeruginosa* infection in a subject.

14. An in vitro method of eliminating or preventing a *Pseudomonas aeruginosa* infection of a cell in culture, said method comprising bringing into contact said cell with a peptide or depsipeptide according to any one of claims 1 to 10.

15. A method for identifying a compound, wherein said method comprises
(a) (aa) bringing into contact *Pseudomonas aeruginosa* ExoS protein or a fragment thereof, said fragment comprising the residues of said ExoS protein binding to a 14-3-3 protein, with said 14-3-3 protein, wherein said ExoS protein or fragment thereof comprises a label L¹ and said 14-3-3 protein comprises a label L²;
(ab) determining fluorescence of said label(s) L¹ and/or L² prior to and after said bringing into contact;
wherein a change in said fluorescence of said label(s) L¹ and/or L² is indicative of a compound which interferes with the binding of said *Pseudomonas aeruginosa* ExoS protein to said 14-3-3 protein;
(b) (ba) bringing into contact said 14-3-3 protein and a cognate endogenous phosphorylated protein or fragment thereof, said fragment comprising the residues of said phosphorylated protein binding to said 14-3-3 protein, wherein said phosphorylated protein or fragment thereof comprises a label L³ and said 14-3-3 protein comprises a label L⁴;
(bb) determining fluorescence of said label(s) L³ and/or L⁴ prior to and after said bringing into contact;
wherein no change in said fluorescence of said label(s) L³ and/or L⁴ is indicative of said compound not interfering with the binding of a cognate endogenous phosphorylated protein to said 14-3-3 protein.


SEQUENCE LISTING

<110> Max-Planck-Gesellschaft zur FÃ¶rderung der Wissenschaften e.V.

<120> Means and methods for treating Pseudomonas infection

<130> W1727 EP

<150> EP 13 17 2741.4
<151> 2013-06-19

<160> 16

<170> BiSSAP 1.3

<210> 1
<211> 246
<212> PRT
<213> Homo sapiens

<220>
<223> sp|P31946|1433B_HUMAN 14-3-3 protein beta

<400> 1
Met Thr Met Asp Lys Ser Glu Leu Val Gln Lys Ala Lys Leu Ala Glu
1               5                   10                  15
Gln Ala Glu Arg Tyr Asp Asp Met Ala Ala Ala Met Lys Ala Val Thr
            20                  25                  30
Glu Gln Gly His Glu Leu Ser Asn Glu Glu Arg Asn Leu Leu Ser Val
        35                  40                  45
Ala Tyr Lys Asn Val Val Gly Ala Arg Arg Ser Ser Trp Arg Val Ile
    50                  55                  60
Ser Ser Ile Glu Gln Lys Thr Glu Arg Asn Glu Lys Lys Gln Gln Met
65                  70                  75                  80
Gly Lys Glu Tyr Arg Glu Lys Ile Glu Ala Glu Leu Gln Asp Ile Cys
                85                  90                  95
Asn Asp Val Leu Glu Leu Leu Asp Lys Tyr Leu Ile Pro Asn Ala Thr
            100                 105                 110
Gln Pro Glu Ser Lys Val Phe Tyr Leu Lys Met Lys Gly Asp Tyr Phe
        115                 120                 125
Arg Tyr Leu Ser Glu Val Ala Ser Gly Asp Asn Lys Gln Thr Thr Val
    130                 135                 140
Ser Asn Ser Gln Gln Ala Tyr Gln Glu Ala Phe Glu Ile Ser Lys Lys
145                 150                 155                 160
Glu Met Gln Pro Thr His Pro Ile Arg Leu Gly Leu Ala Leu Asn Phe
                165                 170                 175
Ser Val Phe Tyr Tyr Glu Ile Leu Asn Ser Pro Glu Lys Ala Cys Ser
            180                 185                 190
Leu Ala Lys Thr Ala Phe Asp Glu Ala Ile Ala Glu Leu Asp Thr Leu
        195                 200                 205
Asn Glu Glu Ser Tyr Lys Asp Ser Thr Leu Ile Met Gln Leu Leu Arg
    210                 215                 220
Asp Asn Leu Thr Leu Trp Thr Ser Glu Asn Gln Gly Asp Glu Gly Asp
225                 230                 235                 240
Ala Gly Glu Gly Glu Asn
                245

<210> 2
<211> 247
<212> PRT
<213> Homo sapiens

<220>

<223> sp|P61981|1433G_HUMAN 14-3-3 protein gamma

<400> 2

<210> 3
<211> 255
<212> PRT
<213> Homo sapiens

<220>
<223> sp|P62258|1433E_HUMAN 14-3-3 protein epsilon

<400> 3
Met Asp Asp Arg Glu Asp Leu Val Tyr Gln Ala Lys Leu Ala Glu Gln
1               5                   10                  15
Ala Glu Arg Tyr Asp Glu Met Val Glu Ser Met Lys Lys Val Ala Gly
            20                  25                  30
Met Asp Val Glu Leu Thr Val Glu Glu Arg Asn Leu Leu Ser Val Ala
        35                  40                  45
Tyr Lys Asn Val Ile Gly Ala Arg Arg Ala Ser Trp Arg Ile Ile Ser
    50                  55                  60
Ser Ile Glu Gln Lys Glu Glu Asn Lys Gly Gly Glu Asp Lys Leu Lys
65                  70                  75                  80
Met Ile Arg Glu Tyr Arg Gln Met Val Glu Thr Glu Leu Lys Leu Ile
                85                  90                  95
Cys Cys Asp Ile Leu Asp Val Leu Asp Lys His Leu Ile Pro Ala Ala
            100                 105                 110
Asn Thr Gly Glu Ser Lys Val Phe Tyr Tyr Lys Met Lys Gly Asp Tyr
        115                 120                 125
His Arg Tyr Leu Ala Glu Phe Ala Thr Gly Asn Asp Arg Lys Glu Ala
    130                 135                 140

Ala Glu Asn Ser Leu Val Ala Tyr Lys Ala Ala Ser Asp Ile Ala Met
145                 150                 155                 160
Thr Glu Leu Pro Pro Thr His Pro Ile Arg Leu Gly Leu Ala Leu Asn
                165                 170                 175
Phe Ser Val Phe Tyr Tyr Glu Ile Leu Asn Ser Pro Asp Arg Ala Cys
            180                 185                 190
Arg Leu Ala Lys Ala Ala Phe Asp Asp Ala Ile Ala Glu Leu Asp Thr
        195                 200                 205
Leu Ser Glu Glu Ser Tyr Lys Asp Ser Thr Leu Ile Met Gln Leu Leu
    210                 215                 220
Arg Asp Asn Leu Thr Leu Trp Thr Ser Asp Met Gln Gly Asp Gly Glu
225                 230                 235                 240
Glu Gln Asn Lys Glu Ala Leu Gln Asp Val Glu Asp Glu Asn Gln
                245                 250                 255

<210> 4
<211> 245
<212> PRT
<213> Homo sapiens

<220>
<223> sp|P63104|1433Z_HUMAN 14-3-3 protein zeta/delta

<400> 4
Met Asp Lys Asn Glu Leu Val Gln Lys Ala Lys Leu Ala Glu Gln Ala
1               5                   10                  15
Glu Arg Tyr Asp Asp Met Ala Ala Cys Met Lys Ser Val Thr Glu Gln
            20                  25                  30
Gly Ala Glu Leu Ser Asn Glu Glu Arg Asn Leu Leu Ser Val Ala Tyr
        35                  40                  45
Lys Asn Val Val Gly Ala Arg Arg Ser Ser Trp Arg Val Val Ser Ser
    50                  55                  60
Ile Glu Gln Lys Thr Glu Gly Ala Glu Lys Lys Gln Gln Met Ala Arg
65                  70                  75                  80
Glu Tyr Arg Glu Lys Ile Glu Thr Glu Leu Arg Asp Ile Cys Asn Asp
                85                  90                  95
Val Leu Ser Leu Leu Glu Lys Phe Leu Ile Pro Asn Ala Ser Gln Ala
            100                 105                 110
Glu Ser Lys Val Phe Tyr Leu Lys Met Lys Gly Asp Tyr Tyr Arg Tyr
        115                 120                 125
Leu Ala Glu Val Ala Ala Gly Asp Asp Lys Lys Gly Ile Val Asp Gln
    130                 135                 140
Ser Gln Gln Ala Tyr Gln Glu Ala Phe Glu Ile Ser Lys Lys Glu Met
145                 150                 155                 160
Gln Pro Thr His Pro Ile Arg Leu Gly Leu Ala Leu Asn Phe Ser Val
                165                 170                 175
Phe Tyr Tyr Glu Ile Leu Asn Ser Pro Glu Lys Ala Cys Ser Leu Ala
            180                 185                 190
Lys Thr Ala Phe Asp Glu Ala Ile Ala Glu Leu Asp Thr Leu Ser Glu
        195                 200                 205
Glu Ser Tyr Lys Asp Ser Thr Leu Ile Met Gln Leu Leu Arg Asp Asn
    210                 215                 220
Leu Thr Leu Trp Thr Ser Asp Thr Gln Gly Asp Glu Ala Glu Ala Gly
225                 230                 235                 240
Glu Gly Gly Glu Asn
                245

<210> 5
<211> 246
<212> PRT
<213> Homo sapiens

<220>

<223> sp|Q04917|1433F_HUMAN 14-3-3 protein eta

<400> 5
Met Gly Asp Arg Glu Gln Leu Leu Gln Arg Ala Arg Leu Ala Glu Gln
1               5                   10                  15
Ala Glu Arg Tyr Asp Asp Met Ala Ser Ala Met Lys Ala Val Thr Glu
            20                  25                  30
Leu Asn Glu Pro Leu Ser Asn Glu Asp Arg Asn Leu Leu Ser Val Ala
        35                  40                  45
Tyr Lys Asn Val Val Gly Ala Arg Arg Ser Ser Trp Arg Val Ile Ser
    50                  55                  60
Ser Ile Glu Gln Lys Thr Met Ala Asp Gly Asn Glu Lys Lys Leu Glu
65                  70                  75                  80
Lys Val Lys Ala Tyr Arg Glu Lys Ile Glu Lys Glu Leu Glu Thr Val
                85                  90                  95
Cys Asn Asp Val Leu Ser Leu Leu Asp Lys Phe Leu Ile Lys Asn Cys
            100                 105                 110
Asn Asp Phe Gln Tyr Glu Ser Lys Val Phe Tyr Leu Lys Met Lys Gly
        115                 120                 125
Asp Tyr Tyr Arg Tyr Leu Ala Glu Val Ala Ser Gly Glu Lys Lys Asn
    130                 135                 140
Ser Val Val Glu Ala Ser Glu Ala Ala Tyr Lys Glu Ala Phe Glu Ile
145                 150                 155                 160
Ser Lys Glu Gln Met Gln Pro Thr His Pro Ile Arg Leu Gly Leu Ala
                165                 170                 175
Leu Asn Phe Ser Val Phe Tyr Tyr Glu Ile Gln Asn Ala Pro Glu Gln
            180                 185                 190
Ala Cys Leu Leu Ala Lys Gln Ala Phe Asp Asp Ala Ile Ala Glu Leu
        195                 200                 205
Asp Thr Leu Asn Glu Asp Ser Tyr Lys Asp Ser Thr Leu Ile Met Gln
    210                 215                 220
Leu Leu Arg Asp Asn Leu Thr Leu Trp Thr Ser Asp Gln Gln Asp Glu
225                 230                 235                 240
Glu Ala Gly Glu Gly Asn
                245

<210> 6
<211> 248
<212> PRT
<213> Homo sapiens

<220>
<223> sp|P31947|1433S_HUMAN 14-3-3 protein sigma

<400> 6

<210> 7
<211> 245
<212> PRT
<213> Homo sapiens

<220>
<223> sp|P27348|1433T_HUMAN 14-3-3 protein theta

<400> 7
Met Glu Lys Thr Glu Leu Ile Gln Lys Ala Lys Leu Ala Glu Gln Ala
1               5                   10                  15
Glu Arg Tyr Asp Asp Met Ala Thr Cys Met Lys Ala Val Thr Glu Gln
            20                  25                  30
Gly Ala Glu Leu Ser Asn Glu Glu Arg Asn Leu Leu Ser Val Ala Tyr
        35                  40                  45
Lys Asn Val Val Gly Gly Arg Arg Ser Ala Trp Arg Val Ile Ser Ser
    50                  55                  60
Ile Glu Gln Lys Thr Asp Thr Ser Asp Lys Lys Leu Gln Leu Ile Lys
65                  70                  75                  80
Asp Tyr Arg Glu Lys Val Glu Ser Glu Leu Arg Ser Ile Cys Thr Thr
                85                  90                  95
Val Leu Glu Leu Leu Asp Lys Tyr Leu Ile Ala Asn Ala Thr Asn Pro
            100                 105                 110
Glu Ser Lys Val Phe Tyr Leu Lys Met Lys Gly Asp Tyr Phe Arg Tyr
        115                 120                 125
Leu Ala Glu Val Ala Cys Gly Asp Asp Arg Lys Gln Thr Ile Asp Asn
    130                 135                 140
Ser Gln Gly Ala Tyr Gln Glu Ala Phe Asp Ile Ser Lys Lys Glu Met
145                 150                 155                 160
Gln Pro Thr His Pro Ile Arg Leu Gly Leu Ala Leu Asn Phe Ser Val
                165                 170                 175
Phe Tyr Tyr Glu Ile Leu Asn Asn Pro Glu Leu Ala Cys Thr Leu Ala
            180                 185                 190
Lys Thr Ala Phe Asp Glu Ala Ile Ala Glu Leu Asp Thr Leu Asn Glu
        195                 200                 205
Asp Ser Tyr Lys Asp Ser Thr Leu Ile Met Gln Leu Leu Arg Asp Asn
    210                 215                 220
Leu Thr Leu Trp Thr Ser Asp Ser Ala Gly Glu Glu Cys Asp Ala Ala
225                 230                 235                 240
Glu Gly Ala Glu Asn
                245

<210> 8
<211> 15
<212> PRT
<213> Artificial Sequence

<220>

<223> subsequence of ExoS

<400> 8
Ser Gly His Gly Gln Gly Leu Leu Asp Ala Leu Asp Leu Ala Ser
1               5                   10                  15

<210> 9
<211> 4
<212> PRT
<213> Artificial Sequence

<220>
<223> definition of moiety (Y)m

<400> 9
Asp Leu Ala Ser
1

<210> 10
<211> 4
<212> PRT
<213> Artificial Sequence

<220>
<223> definition of moiety (Y)m

<400> 10
Asn Leu Ala Ser
1

<210> 11
<211> 11
<212> PRT
<213> Artificial Sequence

<220>
<223> subsequence of ExoS

<400> 11
Gln Gly Leu Leu Asp Ala Leu Asp Leu Ala Ser
1               5                   10

<210> 12
<211> 13
<212> PRT
<213> Artificial Sequence

<220>
<223> subsequence of ExoS

<400> 12
His Ser Gln Gly Leu Leu Asp Ala Leu Asp Leu Ala Ser
1               5                   10

<210> 13
<211> 9
<212> PRT
<213> Artificial Sequence

<220>
<223> subsequence of 14-3-3 used for alignment

<400> 13

Arg Asn Leu Leu Ser Val Ala Tyr Lys
1               5

<210> 14
<211> 4
<212> PRT
<213> Artificial Sequence

<220>
<223> subsequence of 14-3-3 used for alignment

<400> 14
Phe Tyr Leu Lys
1

<210> 15
<211> 4
<212> PRT
<213> Artificial Sequence

<220>
<223> subsequence of 14-3-3 used for alignment

<400> 15
Asp Ser Thr Leu
1

<210> 16
<211> 15
<212> PRT
<213> Artificial Sequence

<220>
<223> subsequence of ExoS

<400> 16
Ser Gly His Ser Gln Gly Leu Leu Asp Ala Leu Asp Leu Ala Ser
1               5                   10                  15